# EUROPÄISCHE PATENTSCHRIFT

(11) **EP 1 054 039 B1**
(45) Veröffentlichungstag und Bekanntmachung des Hinweises auf die Patenterteilung: **25.02.2004**
(21) Anmeldenummer: 00110415.7
(22) Anmeldetag: 16.05.2000
(51) Int. Cl.: C09B 11/24, G01N 33/58, G01N 33/533, C12Q 1/68

(54) **Neue Fluoreszenzfarbstoffe und ihre Verwendung als Fluoreszenzmarker**
Fluorescent dyes and their use as fluorescent labelling agents
Colorants fluorescents et leur utilisation comme marqueurs fluorescents

(30) Priorität: 20.05.1999 DE 19923168
(43) Veröffentlichungstag der Anmeldung: 22.11.2000
(73) Patentinhaber: Roche Diagnostics GmbH, 68298 Mannheim (DE)
(72) Erfinder: Daltrozzo, Ewald, Prof. Dr., 78467 Konstanz (DE); Reiss, Alexander, Dr., 88699 Frickingen (DE)

(56) Entgegenhaltungen:
- EP-A- 0 543 333
- CH-A4- 1 243 370
- US-A- 5 847 162

## Beschreibung

Die beanspruchte Erfindung betrifft die Herstellung neuer Fluoreszenzfarbstoffe, deren Absorptionsbereiche oberhalb 650 nm liegen, die Verwendung dieser Farbstoffe zur Markierung von Biomolekülen und die Verwendung der Marker-Biomolekül-Konjugate in diagnostischen Systemen.

Zur Durchführung immunologischer Assays und in der DNA-Analytik werden Marker oder Label benötigt, die nach Ablauf einer analytspezifischen Reaktion eine Quantifizierung des Analyten erlauben.

Aufgrund der hohen Sensitivität haben sich in der letzten Zeit besonders Fluoreszenzlabel durchgesetzt. So ermöglicht die Markierung eines Antikörpers oder eines Oligo-Nukleotids mit Fluoreszenzfarbstoffen eine direkte Quantifizierung.

Weit verbreitete Fluoreszenzfarbstoffe sind beispielsweise FITC (Fluoreszeinisothiocyanat), FLUOS (Fluorescein N-Hydroxysuccinimidester), Resorufin und Rhodaminlabel, die aber für ihre Anregung relativ aufwendige Lichtquellen, zum Beispiel Argonlaser, benötigen.

Die rasche Entwicklung preiswerter Laserdioden mit einem Emissionsbereich von 630 780 nm, die sich zudem hervorragend zum Aufbau miniaturisierter Systeme eignen, macht Farbstoffe wünschenswert, die in diesen Wellenlängenbereichen absorbieren.

In EP-A-0 543 333 werden pentazyklische Rhodamin-Farbstoffe beschrieben, die als Label eingesetzt werden können. Die Schwerpunkte der Absorption solcher Farbstoffe liegen im Bereich von ≤ 660 nm.

In der WO 88/047 77 werden Phthalocyaninfarbstoffe beschrieben, die allerdings mehr als eine funktionelle Gruppe besitzen, so daß sie bei Konjugation, zum Beispiel mit Antikörpern, zu Vernetzungen und Produktgemischen führen, die einen großen Reinigungsaufwand erfordern.

In DE 195 21231 werden neue Oxazinfarbstoffe und deren Verwendung als Fluoreszenzmarker beschrieben, die ein Absorptionsmaximum bis zu 700 nm besitzen.

CH 12 433 /70 beschreibt ein Verfahren zum Färben oder Bedrucken von Textilmaterial.

US 5,847,162 offenbart 4,7-Dichlororhodemin verbindungen und ihre Verwendung als Fluoreszenzmarker.

Aufgabe der vorliegenden Erfindung ist es, Farbstoffe zur Verfügung zu stellen, die sich für eine Kupplung mit biologischen Molekülen eignen, die über eine hohe Quantenausbeute verfügen, in einem Spektralbereich oberhalb 650 nm absorbieren und eine möglichst geringe unspezifische Bindung an biologische Verbindungen oder an Festphasen aufweisen.

Gelöst wird die Aufgabe durch die Erfindung, wie sie in den Ansprüchen charakterisiert ist.

Gegenstand der Erfindung sind neue Xanthenderivate der allgemeinen Formel I, 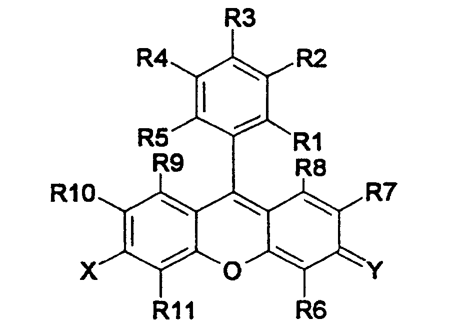 worin R1,R2, R3, R4, R5 unabhängig voneinander Wasserstoff, Alkyl, Aryl, Heteroaryl, Alkoxy, Cyano, Hydroxy, Halogen, Isocyanat, Isothiocyanat oder Carboxyl, Sulfonyl bzw. Derivate davon, wie Alkoxycarbonyl, Aryloxycarbonyl, Aktivester, Säurehalogenide, gemischte Anhydride oder unsubstituierte oder substituierte bzw. unsubsituierte Aminoreste, speziell ω-Aminoalkohole, ω-Aminocarbonsäuren oder ω-Aminohalogenalkane sein können.

Als vorteilhaft hat sich herausgestellt, wenn mindestens ein Rest der Gruppe R1,R2, R3, R4, R5 ein Halogen, insbesondere Fluor oder Chlor, darstellt und mindestens ein Rest der Gruppe R1,R2, R3, R4, R5 eine funktionelle Gruppe ist, bevorzugt eine Säure-Gruppe, besonders bevorzugt eine Carbonsäure oder ein Carbonsäure-Derivat.

Bei den Resten R6, R7, R8, R9, R10, R11 handelt es sich unabhängig voneinander um Wasserstoff, Alkyl-, substituierte Alkylreste oder Halogenid, besonders bevorzugt sind Wasserstoffe oder Alkylreste mit 1-10 C-Atomen, oder Sulfoalkylreste, besonders Sulfomethylreste oder Chlor-Reste und Fluor-Reste.

Mindestens einer der Reste X oder Y ist elektronenziehend. Bevorzugte elektronenziehende Reste sind C-Nukleophile aus aktivierten Methylaromaten oder -heteroaromaten, wie z.B. Heteroarylidencyanmethine vom Typ der 2-Benzthiazolylidencyanmethine bzw. 2- oder 4-Chinolinylidencyanmethine oder Malonsäurederivate, wie 2-Propyldinitril-Reste oder Cyanessigsäureester.

Mindestens ist X oder Y ein solcher Rest, oder X und Y sind solche Reste. Die Reste X und Y können gleich oder verschieden sein.

Desweiteren kann es sich bei X auch um Amin-, bzw. substituierte Amin-Reste handeln, besonders bevorzugt sind Alkyl-substituierte bzw. cyclische Amine, wobei dann Y ausgewählt wird aus der Reihe der elektronenziehenden Reste (insbesondere Heteroarylidencyanmethin-Reste bzw. Malonsäurederivate), wie sie oben beschrieben sind.

Mit den erfindungsgemäßen Verbindungen werden Moleküle bereitgestellt, die sich aufgrund ihrer spektralen Eigenschaften (Absorptionsmaxima im Bereich von ca.650 nm und darüber sowie Emissionsmaxima oberhalb 670 nm) sehr gut als Farbstoffe und insbesondere als Fluoreszenzfarbstoffe eignen. Besonders bevorzugt sind Absorptionsmaxima über 700 nm. Dabei können die spektralen Eigenschaften der Moleküle dadurch verändert werden, daß Identität, Anzahl und Position der Reste bezüglich R1, R2, R3, R4, R5, R6, R7, R8, R9, R10, R11 verändert werden. Auf diese Weise lassen sich Fluoreszenzfarbstoffe mit nahezu beliebigen Absorptionsund Emissionsmaxima oberhalb 650nm herstellen. Ein Gegenstand der Erfindung ist somit auch die Verwendung der erfindungsgemäßen Xanthen-Derivate als Fluoreszenzfarbstoff bzw. als Laserfarbstoff.

In den erfindungsgemäßen Verbindungen der allgemeinen Formel I liegt vorzugsweise zumindest einer der Reste R1, R2, R3, R4, R5 als eine kupplungsfähige, aktivierte Gruppe oder zu einer Kupplung aktivierbaren Gruppe vor. Eine solche aktivierte Gruppe leitet sich insbesondere von einer aktivierbaren Carbonsäure- oder Sulfonsäuregruppierung ab, und ist zum Beispiel ein Säureester, ein Säureanhydrid, ein Säurehalogenid, vorzugsweise Bromid, insbesondere Chlorid oder ein N-Hydroxy-Succinimidester oder ein ω-Alkylhalogenid. Desweiteren kann es sich bei einer solchen aktivierten Gruppe auch z. B. um Phosphoramidite handeln.

Die Tabelle gibt einige Beispiele für aktivierte, kupplungsfähige Gruppen. Dem Fachmann sind weitere solcher Gruppen aus der Synthesechemie für Konjugate bekannt.

**Tabelle 1**

| Aktivierte Gruppe | Verknüpfung mit | Produkt |
|---|---|---|
| NHS-Ester | Amine | Amid |
| Isothiocyanat | Amine | Thioharnstoff |
| (Gemischtes) Anhydrid | Amine | Amide |
| Maleinmid | Thiol | Thioether |
| Thiol | Maleinmid | Thioether |
| Haloacetyl | Thiol | Thioder |
| Hydrazine | Aldehyd | Hydrazone |
| Amine | Aldehyd | Amine (nach reduktion) |
| Amine | Reaktive Carbonsäure | Amide |
| Phosphoramidite | Geschützte Nukleotide | Gelabelte Oligonukleotide |

Zur Herstellung von Konjugaten, welche die erfindungsgemäßen Xanthenderivate enthalten, können aktivierte Derivate synthetisiert werden, die beispielsweise zur Markierung von Biomolekülen oder anderen analytischen Reagenzien geeignet sind. Die Herstellung der aktivierten Derivate erfolgt über mindestens eine der aktivierbaren Gruppen an den Resten R1, R2, R3, R4, R5 bzw. X oder Y, wobei die Aktivierung nach vorbekannten Standardprotokollen erfolgen kann, die dem Fachmann bekannt sind. In der Regel erfolgt die Aktivierung über mindestens eine Hydroxylgruppe, Amino-, Sulfo- .oder Carboxylgruppe von R1, R2, R3, R4 oder R5.

Abhängig vom späteren Verwendungszweck können verschiedene reaktive Gruppen eingeführt werden. Phosphoramidite und H-Phosphonate können beispielsweise von einer Hydroxylgruppe abgeleitet werden. Die Herstellung von Xanthen-Phosphoramiditen bzw. H-Phosphonaten erfolgt somit in der Regel nach vorbekannten Protokollen (Methods in Mol. Biol. Vol 20, "Protocols for Oligonucleotides and Analogs, Synthesis and Properties", S. Agrawal Hrsg., Humana Press Totowa, NJ).

N-Hydroxy-Succininimid (NHS)-Ester werden dagegen in der Regel von einer Carboxylgruppe abgeleitet, Maleinimide von einer Aminogruppe (P.Y. Reddy, Synthesis(1998) 999) oder durch Verlängerung einer aktivierten Carbonsäure mit einem entsprechenden ω-Aminoalkylmaleinimid.

Die Herstellung von NHS-Estern erfolgt dabei vorzugsweise nach dem in EP 0 543 333 beschriebenen Verfahren, wobei die freie Carbonsäure mit NHS in Gegenwart eines Kondensationsreagenzes, wie beispielsweise DCC oder MEI umgesetzt wird. Die Herstellung von Xanthen-Isothiocyanaten erfolgt vorzugsweise durch Reaktion von Aminogruppen mit Thiophosgen (Advanced Organic Chemistry, Mc Graw Hill, 2^{nd} edition, S. 383,1997).

Gegenstand der Erfindung sind somit auch aktivierte Derivate der erfindungsgemäßen Xanthene. Vorzugsweise handelt es sich bei den reaktiven Gruppen der aktivierten Derivate um Phosphoramidit, N-Hydroxy-Succininimid (NHS)-Ester, Maleinimid-Alkylamid, H-Phosphonat oder Isothiocyanat oder ω-Alkylhalogenide.

Gegenstand der Erfindung sind ferner Konjugate, die durch Bindung erfindungsgemäßer Xanthenverbindungen bzw. deren aktivierter Derivate erhältlich sind. Derartige Konjugate bestehen folglich aus mindestens zwei Komponenten, von denen eine Komponente ein erfindungsgemäßes Xanthen-Derivat darstellt. Ausgehend von den aktivierten Derivaten erfolgt die Herstellung der Konjugate nach Standardprotokollen. Die jeweils geeigneten Konjugationsmethoden sind dem Fachmann bekannt.

Die erfindungsgemäßen Konjugate können für analytische Zwecke eingesetzt werden, sobald eine zweite Komponente des Konjugates an einen zu bestimmenden Bindungspartner binden kann, und der gebildete Komplex nach Anregung mit Licht einer geeigneten Absorptionswellenlänge dadurch nachgewiesen wird, daß die emittierte Fluoreszenz des gebildeten Komplexes detektiert wird.

Aufgrund der Absorption im NIR sind die erfindungsgemäßen Verbindungen auch geeignet für in-vivo Anwendungen. Hierzu werden wasserlösliche Derivate der erfindungsgemäßen Farbstoffe und deren Konjugate mit Biomolekülen eingesetzt. Die in-vivo-Messungen erfolgen über eine Bestimmung der Fluoreszenz oder der Absorption.

Besonders geeignet ist der Einsatz der erfindungsgemäßen Konjugate für Analysen zu diagnostischen Zwecken bzw. für Analysen von medizinischem oder biologischem Material. Gegenstand der Erfindung sind daher insbesondere solche Konjugate, die mit einem Biomolekül interagieren können. Dabei handelt es sich in der Regel um Konjugate, die als weitere Komponente in der Regel selbst ein oder mehrere Biomoleküle enthalten.

Bei diesen, in den Konjugaten enthaltenen Biomolekülen kann es sich beispielsweise um einzelsträngige oder doppelsträngige Nukleinsäuren wie DNA, RNA, bzw. Triplexstrukturen oder Nukleinsäure-analoga wie PNA, Oligonukleotide sowie Oligonukleotid-Derivate, aber auch einzelne Nukleotide, Nukleotid-Derivate, Nukleotid-Analoga oder Nukleosid-Triphosphate handeln. Die Markierung derartiger Moleküle erfolgt an der 5'- Position bevorzugt über einen NHS-Ester oder ein Phosphoramidit, an der 3'- Position dagegen bevorzugt über ein Farbstoffsubstituiertes Trägermaterial wie zum Beispiel CPG. Die Markierung an anderen Stellen wie beispielsweise den Nukleinsäure-Basen erfolgt ebenfalls vorzugsweise über einen NHS-Ester.

Im Falle einer Markierung von Proteinen, Proteinkomplexen, Antikörpern oder Aminosäuren wird die Konjugation bevorzugt über NHS-Ester, m-Maleinimide oder Isothiocyanat oder ω-Alkylhalogenide durchgeführt. Beispiele für weitere Konjugatkomponenten sind Vitamine, Steroid-Hormone, Lipidmoleküle sowie Haptene. Darüber hinaus können auch komplexere biologische Strukturen wie Membranfraktionen oder ganze Zellen entsprechend markiert werden.

Eine besondere Ausführungsform der erfindungsgemäßen Konjugate stellen Oligonukleotide dar, die mit einem erfindungsgemäßen Xanthen-Derivat konjugiert sind. Auf diese Weise markierte Oligonukleotide können für vorbekannte Methoden der Detektion und Analyse von Nukleinsäuren, beispielsweise durch in situ Hybridisierung (Meyne and Myzis, Methods Mol. BioL 33, 63-74, 1994) oder auch als Primer bei verschiedenen Sequenzierverfahren zum Einsatz kommen (Sheealy et al., AnaL Chem. 67, 247-251, 1995).

Neben der chemisch-synthetischen Markierung von Nukleinsäuren können mit erfindungsgemäßen Xanthenfarbstoffen markierte Ribonukleosidtriphosphate bzw. Desoxyribonukleosidtriphosphate als Substrate für Polymerasen durch verschiedene enzymatische Reaktionen in Nukleinsäuren eingebaut werden. Dies geschieht für DNA mithilfe von DNA-Polymerasen beispielsweise mit der Methode der Nick Translation (Rigby et al., J. Mol. Biol. 113, S. 237, 1977) oder durch "Random Primed Labeling" (Feinberg and Vogelstein, Anal. Biochem. 137, S. 266, 1984). Im Falle von RNA geschieht dies beispielsweise durch T3, T7 oder SP6 RNA-Polymerasen im Sinne einer Transkription. Eine weitere Methode der Markierung von Nukleinsäuren ist über eine sogenannte "3'-tailing-Reaktion mit Hilfe von terminaler Transferase möglich.

Gegenstand der Erfindung ist somit auch die Verwendung erfindungsgemäßer Konjugate zur Markierung von Nukleinsäuren durch chemische oder enzymatische Methoden sowie die Verwendung erfindungsgemäß markierter Hybridisierungssonden zur Detektion und Analyse von Nukleinsäuren.

Für einen analytischen Nachweis wird das erfindungsgemäße Xanthen-Derivat zunächst mit Licht einer geeigneten Wellenlänge angeregt, wobei als Lichtquelle beispielsweise Laser, Laserdioden oder LEDs verwendet werden. Die Detektion der Fluoreszenz erfolgt je nach Analyt durch Meßmethoden, die dem Fachmann bekannt sind. Beispiele hierfür sind Fluoreszenzmikroskopie für in-situ Methoden oder aber die Detektion der emittierten Strahlung durch geeignete Photodioden.

Alternativ zur direkten Anregung des erfmdungsgemäßen Farbstoffs mit Strahlungsenergie geeigneter Wellenlänge kann die Anregung auch durch einen sogenannten Fluoreszenz-Resonanz-Energie-Transfer erfolgen. Bei diesem Prinzip wird ein zweiter Fluoreszenzfarbstoff mit Licht einer geeigneten Wellenlänge angeregt. Aufgrund der räumlichen Nähe der beiden Farbstoffe findet daraufhin ein strahlungsloser Energie-Transfer auf das erfindungsgemäße Xanthen-Derivat statt (Van der Meer et al., Resonance Energy Transfer, VCH, 1994). Die Detektion des von diesem Molekül bei einer bestimmten Wellenlänge emittierten Lichtes kann bevorzugt der quantitativen Bestimmung des Analyten dienen. Gegenstand der Erfindung ist somit auch die Verwendung eines erfindungsgemäßen Xanthen-Derivates bzw eines entsprechenden Konjugates als Bestandteil eines Floureszenz-Resonanz-Energie-Transfer-Systems.

Vorzugsweise dienen dabei die erfindungsgemäßen Verbindungen als Resonanz-Energie-Akzeptoren. Bevorzugte Resonanz-Energie-Donoren für alle erfindungsgemäßen Verbindungen, sind spektral geeignete Fluoreszenzfarbstoffe-Konjugate. Gegenstand der Erfindung ist somit auch die Verwendung erfindungsgemäßer Xanthenderivate bzw. entsprechender Konjugate zusammen mit geeigneten Fluoreszenz-Resonanz-Donor-Konjugaten als Fluoreszenz-Resonanz-Energie-Akzeptor in einem Fluoreszenz-Resonanz-Energie-Transfer-System.

Für den quantitativen Nachweis von Nukleinsäuren sind mit Fluoreszenzfarbstoffen markierte Hybridisierungssonden wie beispielsweise Oligonukleotide geeignet, die nach dem Prinzip des Fluoreszenz-Resonanz-Elektronen-Transfers (FRET) detektiert werden können. Beispielsweise kann im Falle von Oligonukleotiden das 5' Ende mit jeweils einer Farbstoff-Komponente des FRET Systems sowie das 3' Ende mit der jeweils anderen Farbstoff-Komponente des FRET Systems markiert sein. Die Oligonucleotide können dabei jeweils auch innerhalb der Sequenz markiert sein.

In einer bestimmten Ausführungsform wird ein solches mit 2 Farbstoffen markiertes Oligonukleotid während einer Nukleinsäureamplifikation zur Detektion der entstehenden Produkte eingesetzt, wobei die Emission des Fluoreszenz-Resonanz-Energie-Donors bestimmt wird. Ist das Oligonukleotid nicht an das Target gebunden, so ist aufgrund des strahlungsfreien Energietransfers keine Fluoreszenz des Donors meßbar. Bindet jedoch das Oligonukleotid an die Target DNA, so werden aufgrund von Exonukleaseaktivitäten der verwendeten DNA-Polymerase die zwei Farbstoffkomponenten räumlich voneinander getrennt, sodaß eine Fluoreszenz des FRET-Donors meßbar wird (US 5,210,015).

In anderen bevorzugten Ausführungsformen befinden sich die verschiedenen Farbstoffe an zwei verschiedenenen Hybridisierungssonden, die in räumlicher Nähe an die Target-Nukleinsäure hybridisieren können. Dabei kann es sich beispielsweise um zwei Oligonukleotid-Sonden handeln, die an den gleichen Strang der Target-Nukleinsäure hybridisieren, wobei der eine Farbstoff sich am 3'-terminalen Nukleotid der ersten Sonde befindet und der andere Farbstoff sich am 5' terminalen Nukleotid der zweiten Sonde befindet, sodaß die Distanz zwischen beiden nur eine geringe Anzahl von Nukleotiden beträgt, wobei diese Anzahl zwischen 0 und 30 liegen kann. Bei Verwendung von Fluorescein in Kombination mit einem erfindungsgemäßen Xanthen-Derivat haben sich Abstände von 0-15, insbesondere 1-5 Nukleotiden und in vielen Fällen von einem Nukleotid als vorteilhaft herausgestellt. Unter Wahrung der Nukleotid-Abstände zwischen den Farbstoffkomponenten können auch Sonden verwendet werden, die nicht terminal, sondern intern mit einem der Farbstoffe konjugiert sind. Bei doppelsträngigen Target-Nukleinsäuren können auch Sonden eingesetzt werden, die an verschiedene Stränge des Targets binden, sofern ein bestimmter Nukleotidabstand von 0 bis 30 Nukleotiden zwischen den beiden Farbstoff-Komponenten besteht.

Gegenstand der Erfindung ist somit ferner die Verwendung eines erfindungsgemäßen Konjugates bestehend aus einem Oligonukleotid und einem erfindungsgemäßen Xanthen-Derivat zur Analyse von Nukleinsäuren, wobei ein weiteres Konjugat, bestehend aus einem zweiten Oligonukleotid und einem weiteren geeigneten Fluoreszenzfarbstoff eingesetzt wird und nach Anregung eines Farbstoffs, welcher vorzugsweise an das zweite Oligonukleotid gekoppelt ist, ein Fluoreszenz-Resonanz-Energie-Transfer stattfinden kann. Entsprechend markierte Oligonukleotidkombinationen werden im Folgenden als "FRET-Paare" bezeichnet.

Als besonders vorteilhaft hat sich der Einsatz derartiger FRET-Paare zum Nachweis von Amplifikationsprodukten während oder nach einer Polymerase-Kettenreaktion herausgestellt. Gegenstand der Erfindung ist deshalb ferner die Verwendung eines erfindungsgemäßen Konjugates als Bestandteil eines FRET-Paares zur Detektion von Reaktionsprodukten einer Nukleinsäure-Amplifikationsreaktion. In einer besonderen Ausführungsform kann einer der beiden Amplifikationsprimer zugleich mit einem der beiden eingesetzten Farbstoffe markiert sein und somit eine der beiden Komponenten des FRET beisteuern.

Der Einsatz von geeigneten FRET-Paaaren zur Detektion der Amplifikationsprodukte ermöglicht ein sogenanntes "Real-Time Monitoring" von PCR-Reaktionen, wobei Daten zur Generierung des Amplifikationsprodukts in Abhängigkeit von der Zahl der durchlaufenen Reaktionszyklen ermittelt werden können. Dies geschieht in der Regel dadurch, daß aufgrund der Reaktions- und Temperaturbedingungen während des notwendigen Annealings der Amplifikationsprimer auch die Oligonukleotide des FRET-Paares an die Target-Nukleinsäure hybridisieren und bei geeigneter Anregung ein entsprechend meßbares Fluoreszenzsignal emittiert wird. Aufgrund der erhaltenen Daten kann somit die Menge der ursprünglich eingesetzten Target-Nukleinsäure quantitativ bestimmt werden. Deshalb kommt derartigen Ausführungsformen eine besondere Bedeutung bei quantitativen RT-PCR Experimenten zu, bei denen RNA-Konzentrationen einer biologischen Probe quantifiziert werden. Gegenstand der Erfindung ist somit auch die Verwendung von erfindungsgemäßen Konjugaten als Bestandteil eines FRET-Paares zur Detektion von Reaktionsprodukten einer Nukleinsäure-Amplifikationsreaktion, wobei das Reaktionsprodukt in jedem Zyklus detektiert wird. Gegenstand der Erfindung ist ferner die Verwendung von erfindungsgemäßen Konjugaten als Bestandteil eines FRET-Paares zur Durchführung einer quantitative Bestimmung der zu amplifizierenden Nukleinsäure.

In einer anderen Ausführungsform erfolgt die Detektion des Amplifikationsproduktes nach Beendigung der Amplifikationsreaktion, wobei nach Hybridisierung des FRET-Paares an die zu detektierende Target-Nukleinsäure die Temperatur im Rahmen einer Schmelzkurvenanalyse wiederum kontinuierlich erhöht wird. Gleichzeitig wird die in Abhängigkeit von der Temperatur emittierte Fluoreszenz bestimmt. Auf diese Weise werden Sequenzen detektiert, die aufgrund bestimmter Mismatches weniger stringent mit dem eingesetzten FRET-Paar hybridisieren. Die auf diese Weise ermittelten Schmelzpunkte dienen so dem Nachweis von Punktmutationen oder anderen Polymorphismen. Gegenstand der Erfindung ist deshalb auch die Verwendung von erfindungsgemäßen Konjugaten als Bestandteil eines FRET-Paares zur Bestimmung von Schmelzkurven, insbesondere bei der Identifikation von Polymorphismen und Punktmutationen.

Solche Fluoreszenz Resonanz Energie Transfer Prozesse lassen sich generell zur Bestimmung von Molekül-Molekül-Interaktionen verwenden, wie z.B. Protein-Protein Wechselwirkung oder Antigen-Antikörper-Reaktionen. Ein besonderer Vorteil ist die Reaktion unter homogenen Bedingungen.

Als Gegenion läßt sich jedes zur Ladungsneutralisierung geeignete und mit dem je nach pH vorliegenden anionischem Grundgerüst kompatible Kation verwenden.

Bei der Synthese der erfindungsgemäßen Verbindungen der Formel I handelt es sich um den Ersatz nukleofuger Gruppen in 3',6'-Positüon von Xanthenen oder Fluoranen durch C-Nukleophile, die aus CH-aciden Verbindungen mit Basen erhalten werden können. Als nukleofuge Abgangsgruppen können die Halogene (Fluor, Chlor, Brom, Jod), SO₂R- bzw. SO₃R-Gruppen (R = alkyl, aryl), Phosphite etc., als C-Nukleophile, die mit Basen herstellbaren Derivate (Anionen) CH-acider Verbindungen, wie durch elektronenziehende Substituenten aktivierte Methylaromaten bzw. Methylheteroaromaten oder Malonsäurederivate eingesetzt werden. Bevorzugt werden 3',6'-Dichlorfluorane, die aus 3',6'-Dihydroxyfluoranen mit POCl₃/PCl₅ gem. Literatur (A.v.Bayer, Liebigs Ann. 183(1876)18) zugänglich sind, mit den mit LDA (Lithiumdiisopropylamid) erhältlichen Derivaten CH-acider Verbindungen umgesetzt.

Die Ausgangsverbindungen werden bevorzugt so gewählt, daß das Syntheseprodukt eine aktivierbare Gruppe enthält. Solche aktivierbaren Gruppen werden nach bekannten Methoden zu kupplungsfähigen Gruppen aktiviert und mit reaktiven Gruppen biologisch aktiver Moleküle zu Biomolekül-Farbstoff-Konjugaten gekuppelt. Dabei können zwischen den aktivierten Gruppen und den biologisch aktiven Molekülen noch Linker eingebaut werden.

Linker dienen dazu den Abstand zwischen den erfindungsgemäßen Farbstoffen und den Biomolekülen zu variieren. So kann zum Beispiel Aminocapronsäure zur Verlängerung an eine Carbonsäure eingeführt werden. Auch kann damit eine Änderung der Funktionalität bewirkt werden, wie zum Beispiel durch Reaktion einer Carbonsäure mit einem Maleinimidoalkylamin. Desweiteren kann über Ladungen im Linker auch zusätzlich die Löslichkeit des Farbstoffes beeinflußt werden. besonders geeignet sind hierfür Aminosäuren mit Ladungsträgern, wie Lysin oder Glutaminsäure.

Mit den erfindungsgemäßen Verbindungen werden neue Verbindungen bereitgestellt, die sich aufgrund ihrer spektroskopischen Eigenschaften (Absorptionsmaximum oberhalb 650 nm) sehr gut für kupplungsfähige Absorptionsfarbstoffe, insbesondere Fluoreszenzfarbstoffe für die Anwendung in Hapten-, Antikörper-, Protein-Konjugaten, zur Polynukleotidmarkierung und zur Anfärbung von Latices (Fluoreszenzlatices) eignen.

Für den Einbau in Latices sind besonders solche Farbstoffe der erfindungsgemäßen Xanthen-Derivate geeignet, die sich in organischen/nicht wassermischbaren Lösungsmitteln lösen und die durch ein Quellverfahren in Latices eingebaut werden können. Solche fluoreszierende Latices, die einen Durchmesser von ca. 50 nm bis zu mehreren µm haben können, können mittels verschiedener Beschichtungsmethoden z.B. mit Proteinen, Haptenen oder Nucleinsäuren beladen werden.

Geeignet sind auch Farbstoffmischungen, welche mindestens einen erfindungsgemäßen Farbstoff enthalten, so daß innerhalb der einzelnen Latexpartikel ein Resonanz-Energy-Transfer stattfinden kann.

Partikel mit gleicher Absorptionslänge und unterschiedlichen Emissionswellenlängen eignen sich für Multiparameter-Analysen, d.h. für die Bestimmung verschiedener Analyte in einer Probe.

Für die Anwendung in Hapten- / Antikörper- /Protein- oder Polynukleotidkonjugaten ist es vorteilhaft, wenn die Farbstoffe gut wasserlöslich sind. Für diesen Verwendungszweck werden deshalb vorzugsweise Verbindungen der allgemeinen Formel I eingesetzt, in denen R1, R2, R3, R4, R5, R6, R7, R8, R9, R10, R11 möglichst hydrophil sind. Vorzugsweise sind diese Verbindungen substituierte Produkte, die zum Beispiel Carboxyl- oder/und Sulfonsäuregruppen enthalten. Die Kupplung zum Konjugat erfolgt über mindestens einen der aktivierten Substituenten der Reste R1, R2, R3, R4, oder R5 und insbesondere über eine Hydroxysuccinimidgruppierung.

Konjugate der Fluoreszenzfarbstoffe mit Haptenen, wie zum Beispiel Theophylin, Digoxin, T3, T4 oder Protein, wie zum Beispiel Antikörper, eignen sich zum Beispiel zum Einsatz in diagnostischen Systemen, insbesondere für Fluoreszenzimmunoassays oder Fluoreszenzpolarisationsimmunoassays.

Ein weiterer Gegenstand der vorliegenden Erfindung ist ein Verfahren zur Bestimmung einer ersten immunologisch bindefähigen Substanz, das dadurch gekennzeichnet ist, daß ein Konjugat einer erfindungsgemäßen Verbindung mit einer zweiten (immunologisch) bindefähigen Substanz, die gleich oder verschieden mit der ersten Substanz sein kann, verwendet wird und daß die durch eine (immunologische) Bindungsreaktion, welche für die erste Substanz spezifisch ist, verursachte Absorptions- oder Fluoreszenzänderung oder Fluoreszenzpolarisationsänderung der erfindungsgemäßen Verbindung als Maß für die Menge der in der Probe enthaltenden zu bestimmenden Substanz bestimmt wird.

Ein weiterer Gegenstand der Erfindung ist die Verwendung der erfindungsgemäßen Konjugate für Immunoassays.

Im Folgenden ist an einer Reihe von konkreten Beispielen die Synthese der erfindungsgemäßen Xanthenderivate aufgezeigt.

Ist die Grundstruktur ein Fluoran, so liegt die Carboxylfunktion in Position R1 der Formel I als Lactonring vor. Alle anderen Reste R2 bis R11 entsprechen den in Anspruch 1 ausgeführten Definitionen. Wie dem Fachmann bekannt ist, läßt sich die Fluoranform, welche durch einen geschlossenen Lactonring am Rest R1 gekennzeichnet ist, durch Ringöffnung in die Xanthenform überführen. Die Erfindung deckt beide Grundstrukturen ab.

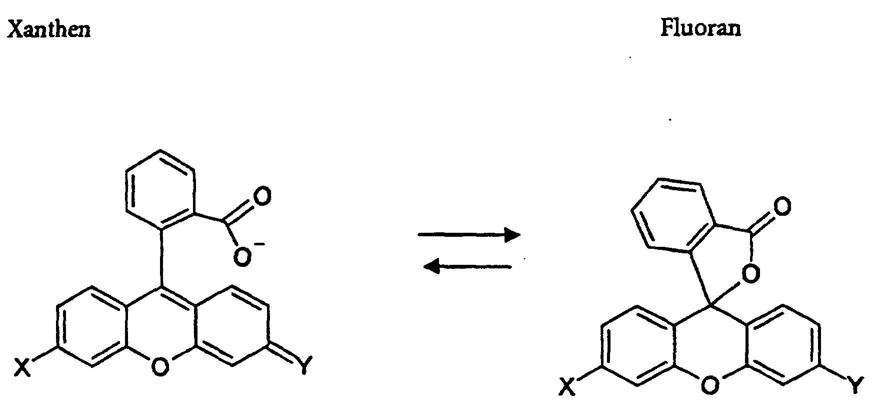

Für die Synthese einer Reihe von Substanzklassen (2, 4, 6, 8) gelten die folgenden allgemeinen Arbeitsbedingungen, welche fallweise auch für die Synthese anderer Substanzklassen zum Tragen kommen. (A) Unter Schutzgasbedingungen wird aus Diisopropylamin und n-Butyllithium eine 0,1 M Lösung von LDA (Lithiumdiisopropylamid) in Dioxan bereitet. Man kühlt auf etwa 7° C ab und gibt die äquimolare Menge einer CH-aciden Komponente zu. Diese Mischung wird bezeichnet als LDA-CH-acid. Zu der so hergestellten Mischung LDA-CH-acid wird das 3',6'abgangsgruppensubstituierte Fluoranderivat erivat (0,4 Mol je Mol LDA) gegeben und sechs Stunden unter Rückfluß erwärmt. Anschließend arbeitet man gemäß Aufarbeitungs-Methode 1 bzw. 2 auf.

### Methode 1:

Die Reaktionslösung wird im Vakuum zur Trockne eingeengt und der Rückstand in Methanol aufgenommen. Die Lösung wird mit Essigsäure bis zum Farbumschlag angesäuert und das Lösungsmittel im Vakuum abdestilliert. Der verbleibende Rückstand wird mit Wasser gewaschen und säulenchromatographisch gereinigt.

### Methode 2:

Die Reaktionslösung wird heiß abgesaugt und der Filterrückstand zunächst mit heißem Dioxan, dann mit Diethylether gewaschen. Man löst den verbliebenen Rückstand in Methanol oder Wasser und säuert mit Mineralsäure bis zum deutlichen Farbumschlag an. Das Lösungsmittel wird im Vakuum abdestilliert und das so erhaltene Rohprodukt mit Wasser gewaschen. Anschließend wird gegebenenfalls durch Säulenchromatographie gereinigt.

Konkrete Ausführungsbeispiele für die Substanzen 2a, 6e und 8a finden sich in den Beispielen 1 bis 3. Andere Beispiele aus diesen Substanzklassen sind den Tabellen 2, 6 und 8 zu entnehmen.

Für die Substanzklasse 3 - welche zwei unterschiedliche elektronenziehende Gruppen aufweist - gibt es ebenfalls eine allgemein gültige Vorgehensweise. Man verfährt wie unter (A) beschrieben, arbeitet allerdings mit einem äquimolaren Verhältnis einer ersten LDA-CH-acid-Mischung und dem 3',6'-abgangsgruppensubstituierten Fluoranderivat. Aufgearbeitet wird, wie unter Methode 1 beschrieben.
Das so erhaltene Produkt wird erneut mit der äquimolaren Menge des Lilthiumsalzes einer zweiten LDA-CH-acid-Mischung in Dioxan umgesetzt. Dieses Lithiumsalz wird aus einer zweiten CH-aciden Komponente und LDA erhalten. Anschließend wird erneut nach der Methode 1 aufgearbeitet.

In Beispiel 4 wird diese Methode anhand von Substanz 3 b exemplarisch für diese Substanzklasse (vergl. Tabelle 3) gezeigt.

Für die Substanzklasse 1 wird zunächst wie unter (A) beschrieben vorgegangen und das so erhaltene Zwischenprodukt isoliert.

Dieses wird anschließend, je nach Siedepunkt des Amins, mit oder ohne Lösungsmittel bei erhöhter Temperatur (ca. 100° C) umgesetzt Die Reaktionslösung wird dann auf Eis/Wasser gegeben und mit Salzsäure angesäuert. Dabei fällt das Produkt aus, man saugt ab und reinigt säulenchromatographisch.

Beispiel 5 stellt exemplarisch anhand der Substanz 1 a das generelle Vorgehen für die Substanzklasse 1 dar (vergl. auch Tabelle 1).

Die angegebenen Verbindungen der Klassen 1, 2, 3, 4, 6 und 8 können z.B. mit Dicyclohexylcarbodiimid in Pyridin mit verschiedenen Alkoholen verestert werden. So können z.B. die Substanzklassen 5, 7 und 9 erhalten werden.

### Beispiel 1:

### Synthese von 3',6'-Bis-(2-benzthiazolyliden-cyanmethin)-fluoran 2a

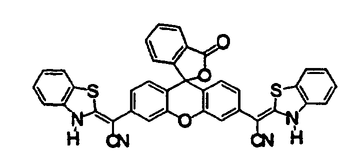

Ansatz: 10,0 g 3',6'-Dichorfluoran (0,027 mol)
19,0 g 2-Cyanmethylbenzthiazol [2*95 g] (0,109 mol)
11,6 ml t-Butylamin [2*58 ml] (0,11 mol)
11,0ml n-Butyllithium, 10M in Hexanfraktion [2*55 ml](0,110mmol)
Durchführung: Methode 2
Reinigung: Die Substanz fällt bereits mit hoher Reinheit an
Ausbeute: 13,6 g = 0,021 mol = 78% d. Th.
Analyse:

| C₃₈H₂₀N₄O₃S₂ | | M_{G} = 644,74 g/mol | |
|---|---|---|---|
| Ber | 70,79% C | 3,13% H | 8,69% N |
| Gef | 70,74% C | 3,23% H | 8,43% N |

Schmelzpunkt: 210°C (Z)
¹H-NMR: 400MHz LM: CD₃OD + CH₃ONa

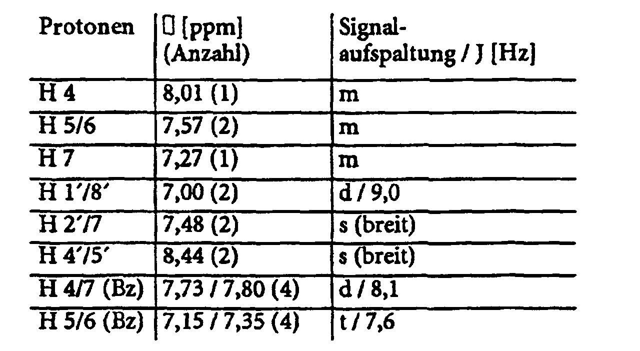

FT-IR (KBr): 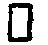 [cm⁻¹] = 2185 (C≡N), 1768 (C=O), 1598,1545,1470, 1420, 1328, 1253, 1082, 883
EI-MS: (70eV; 280°C) [M^{+•}] = 644
UV/VIS: Dianion: Zusatz Triethylamin
Kation: Zusatz: konz. Schwefelsäure

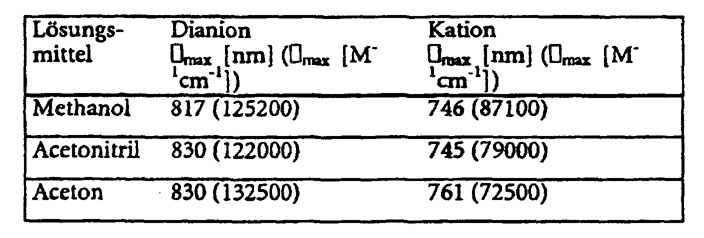

### Beispiel 2:

### Synthese von 5,6,2',7'-Tetrachlor-3',6'-bis-(2-propyl-dinitril)-fluoran 6e

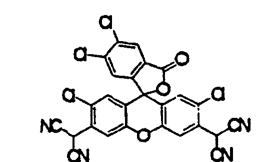

Ansatz: 3,5 g 5,6,3',6'-Tetrachlorfluoran (8,00 mmol)
1,6 g Malonsäuredinitril (24,21 mmol)
2,4 ml n-Butyllithium, 10M in Hexanfraktion (24,00 mmol)
Durchführung: Methode 2
Reinigung: Chromatographie an Kieselgel (Trockensäule) EE/PE 3:2
Ausbeute: 2,95 g = 5,21 mmol = 65% d. Th.
Analyse:

| C₂₆H₈Cl₄N₄O₃ | | M_{G} = 566,19 g/mol | |
|---|---|---|---|
| Ber | 55,16% C | 1,42% H | 9,90% N |

¹H-NMR: 400MHz LM: DMSOd₆

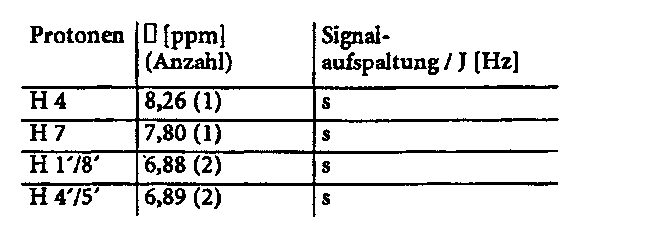

FT-IR (KBr): ν[cm⁻¹] = 2189 (C≡N); 1633; 1569; 1461; 1321; 1203
UV/VIS: Dianion: Zusatz Triethylamin
Anion: Zusatz: Essigsäure

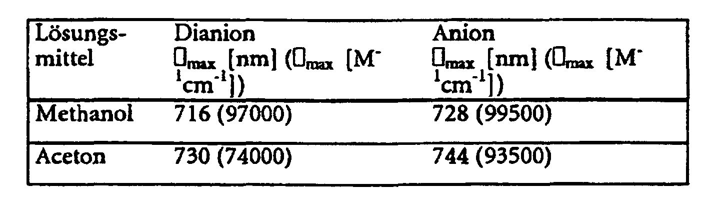

### Beispiel 3:

### Synthese von 3',6'-Bis-(cyanessigsäure-ethylester-2-yl)-fluoran 8a

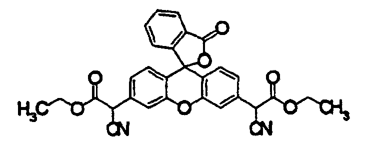

Ansatz: 5,0 g 3',6'-Dichlorfluoran (13,54 mmol)
4,1 ml Cyanessigsäureethylester (38,49 mmol)
5,5 ml Diisopropylamin (39,03 mmol)
3,9 ml n-Butyllithium, 10M in Hexanfraktion (39,00 mmol)
Durchführung: Methode 2
Reinigung: Chromatographie an Kieselgel (Trockensäule) EE/PE 1:1
Ausbeute: 4,53 g = 8,67 mmol = 64 % d. Th.
Analyse:

| C₃₀H₂₂N₂O₇ | | M_{G} = 522,54 g/mol | |
|---|---|---|---|
| Ber | 68,95% C | 4,25% H | 5,36% N |
| Gef | 68,51% C | 4,38% H | 5,47% N |

¹H-NMR: 400MHz LM: CDCl₃

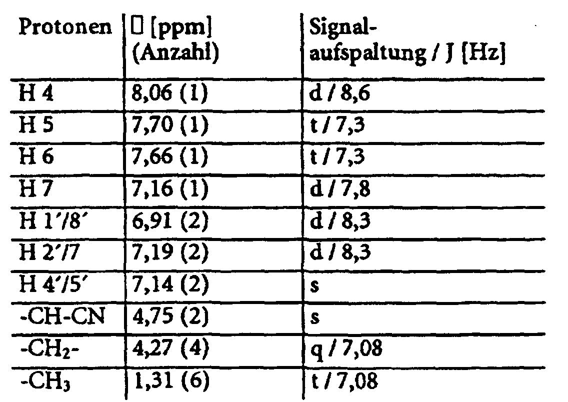

EI-MS: (70 eV; 260°C) [M^{+•}] = 522
UV/VIS: 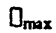 = 695 nm; 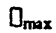 = 105000 M⁻¹cm⁻¹ (Aceton, Zus. Triethylamin)
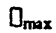 = 676 nm; 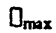 = 119500 M⁻¹cm⁻¹ (Methanol, Zus. Triethylamin)

### Beispiel 4:

### Synthese von 3'-(2-Benzthiazolyliden-cyanmethin)-3'-(2-chinolinyliden-cyanmethin)-4,5,6,7-tetrachlorfluoran 3b

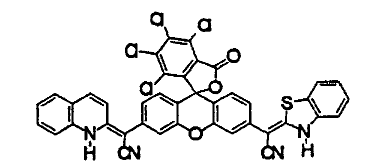

Ansatz: 4,00 g 3'-(2-Chinolinylid-cyanmethylen)-4,5,6,7,6'-pentachlorfluoran (6,26 mmol)
1,20 g 2-Cyanmethylbenzthiazol (6,89 mmol)
0,97 ml Diisopropylamin (6,86 mmol)
0,70 ml n-Butyllithium in Hexanfraktion, 10M (7,00 mmol)
Reinigung: Chromatographie an Kieselgel, Trockensäule EE/PE 1:3
Ausbeute: 1,85 g = 2,38 mmol = 38% d. Th.
Analyse:

| C₄₀H₁₈Cl₄N₄O₃S | | M_{G} = 776,49 g/mol | |
|---|---|---|---|
| Ber | 61,87% C | 2,34% H | 7,22% N |
| Gef | 61,62% C | 2,54% H | 8,04% N |

UV/VIS: 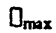 = 859 nm; 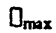 = 40500 M⁻¹cm⁻¹ (Methanol, Zusatz ^{t}BuOK)
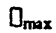 = 867 nm; 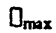 = 37500 M⁻¹cm⁻¹ (Acetonitril, Zusatz TEA)

### Beispiel 5:

### Synthese von 3'-(2-Benzthiazolyliden-cyanmethin)-6'-(N-piperidinyl)-fluoran 1a:

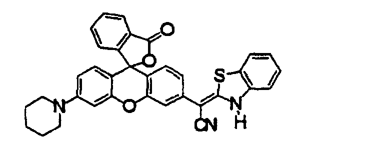

Ansatz: 2,0 g 3'-(2-Benzthiazolylid-cyanmethylen)-6'-chlorfluoran (3,95 mmol)
Reinigung: Chromatographie an Kieselgel (Trockensäule) EE/PE 2:1
Ausbeute: 1,95 g = 3,51 mmol = 89%
Analyse:

| C₃₄H₂₅N₃O₃S | | M_{G} = 555,66 g/mol | |
|---|---|---|---|
| Ber | 73,49% C | 4,54% H | 7,56% N |
| Gef | 73,03%C | 4,82% H | 7,64% N |

EI-MS: (70eV / 300°C) [M^{+•}] = 555
UV/VIS:

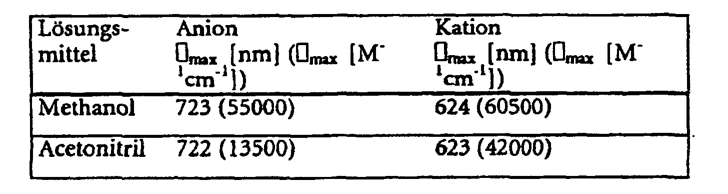

Weitere Beispiele für synthetisierte Derivate sind in den Tabellen 1 - 9 enthalten.

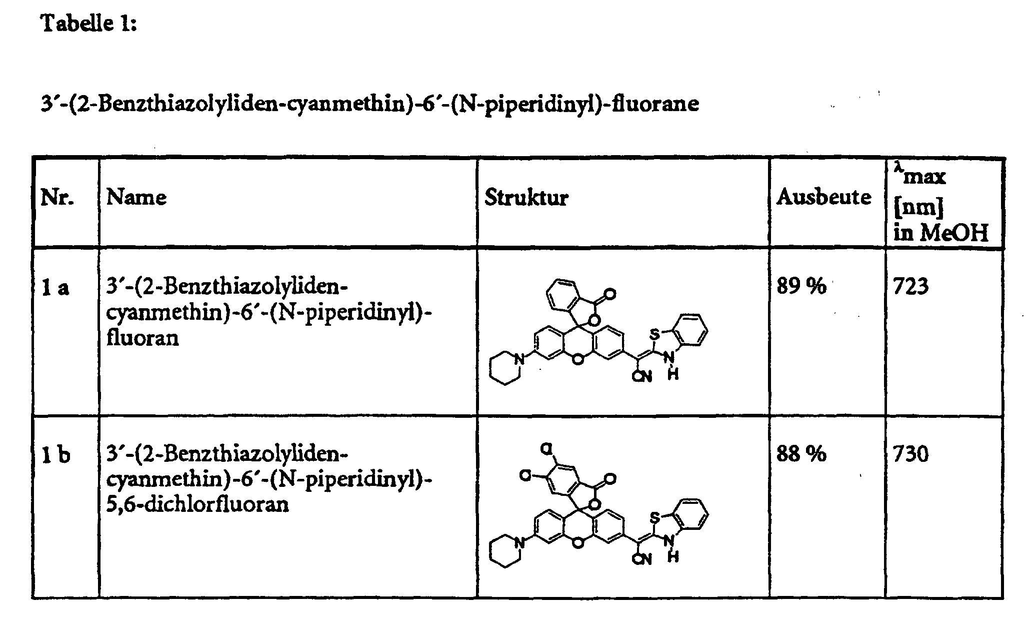

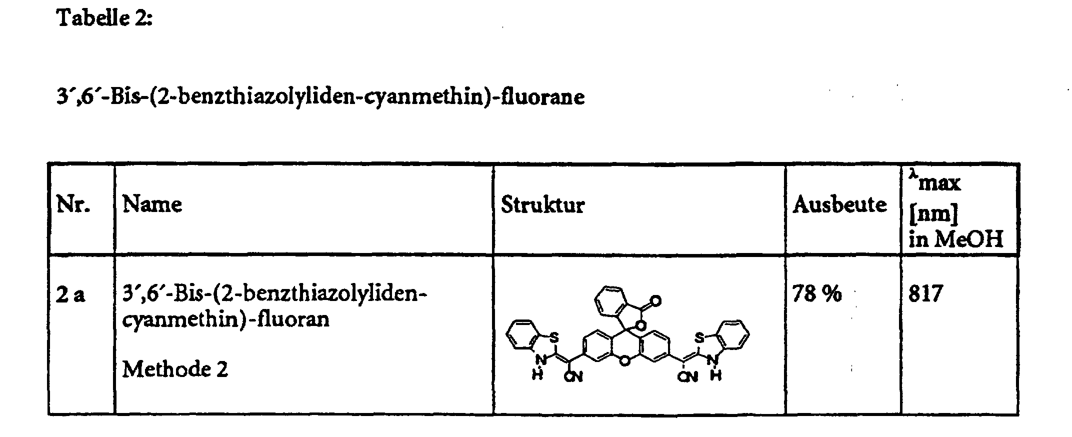

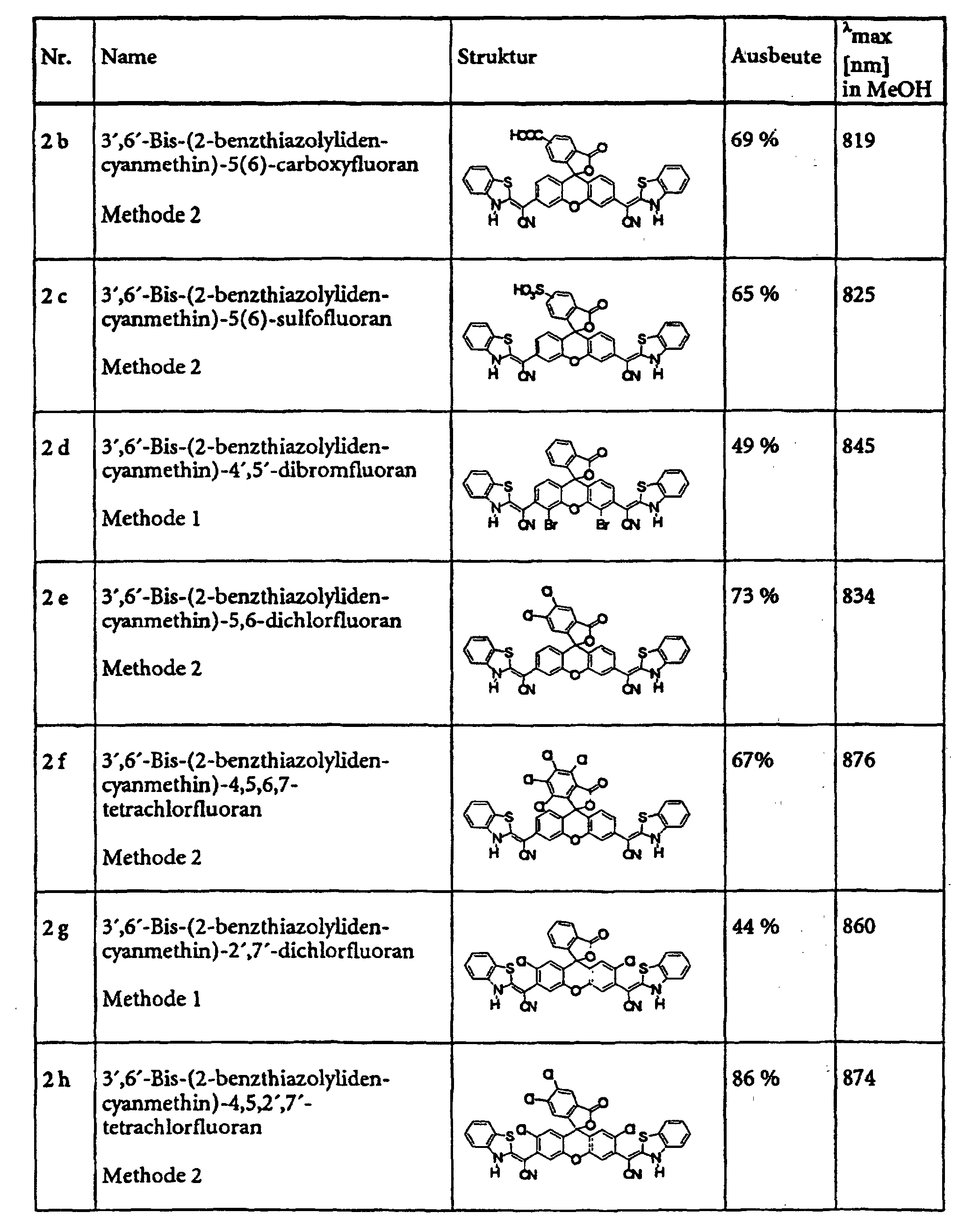

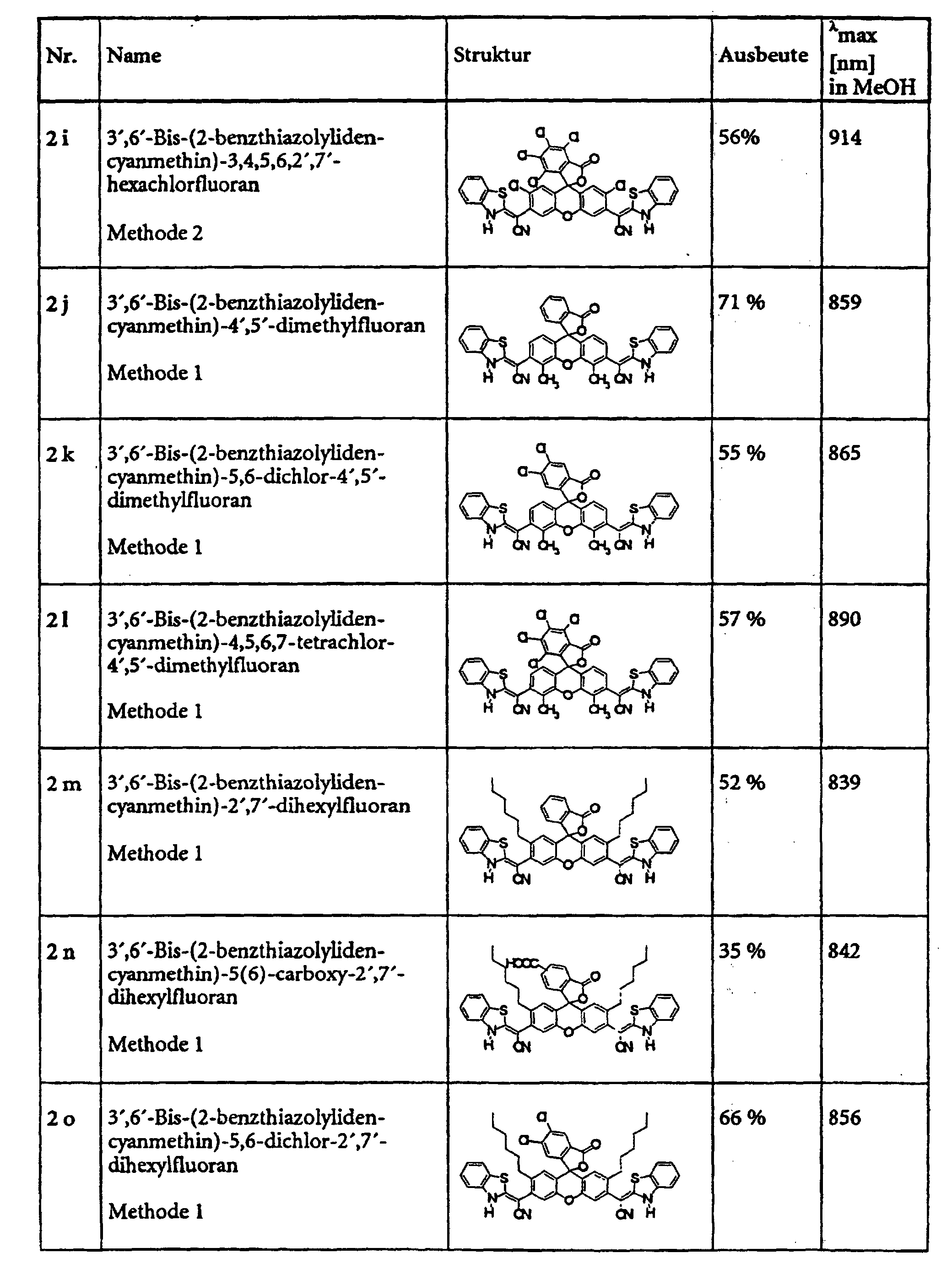

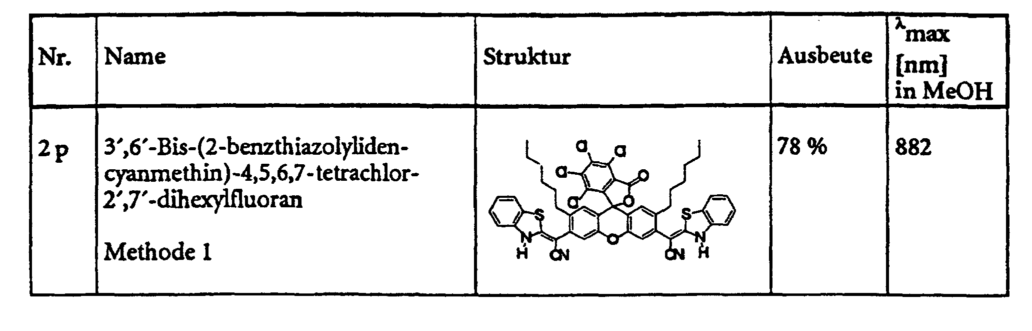

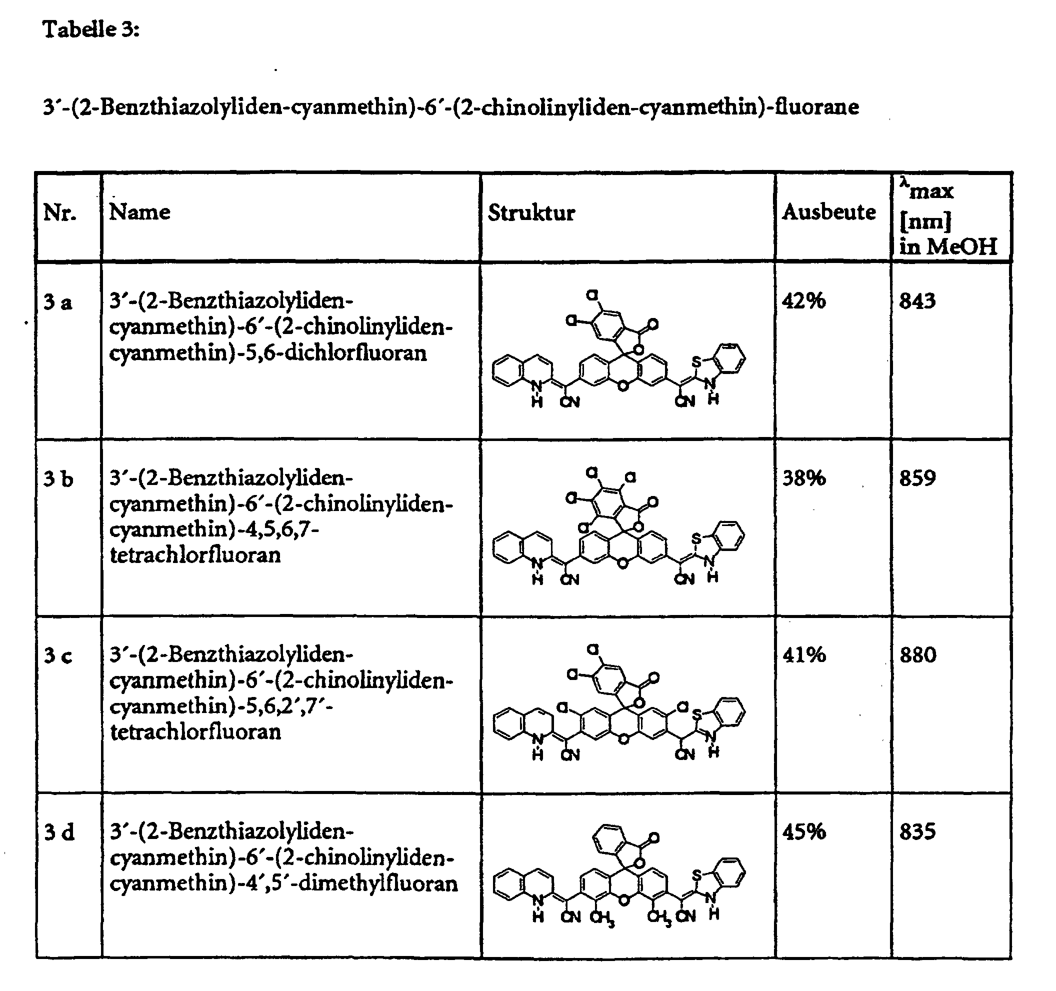

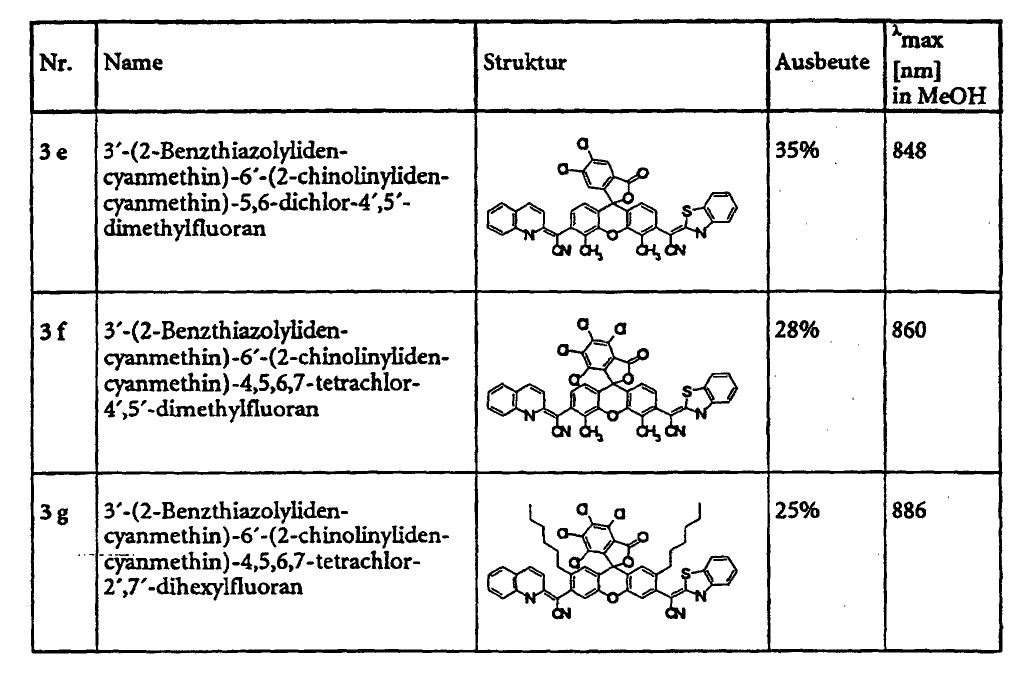

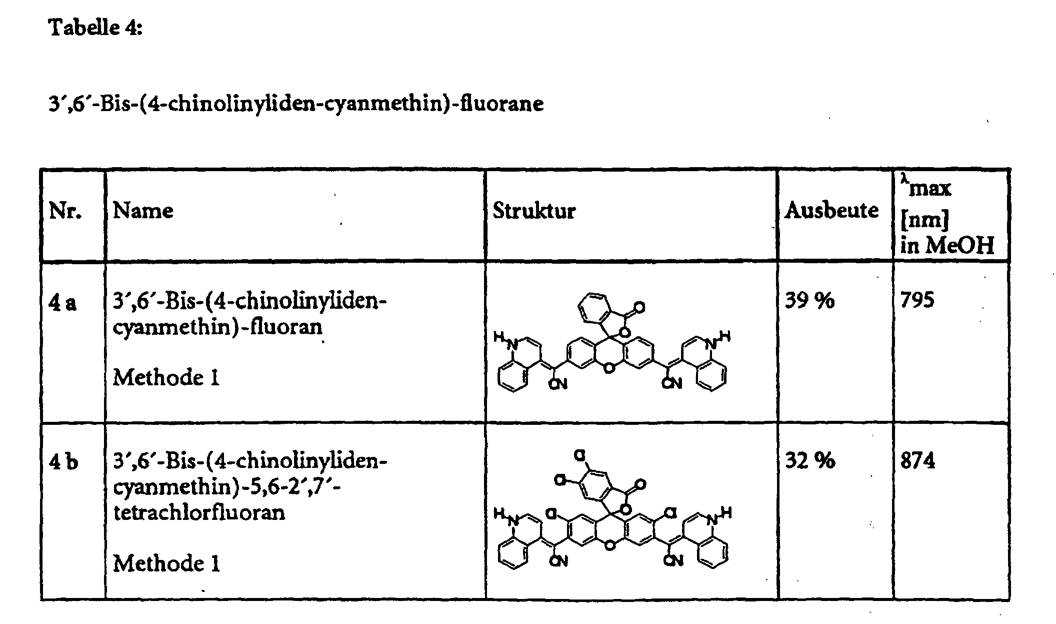

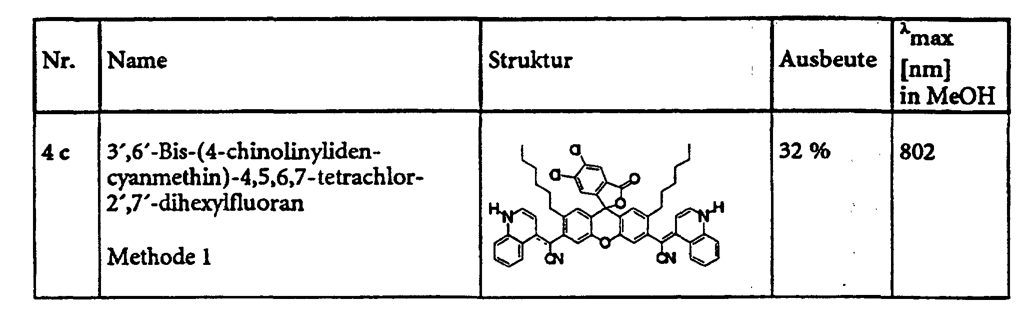

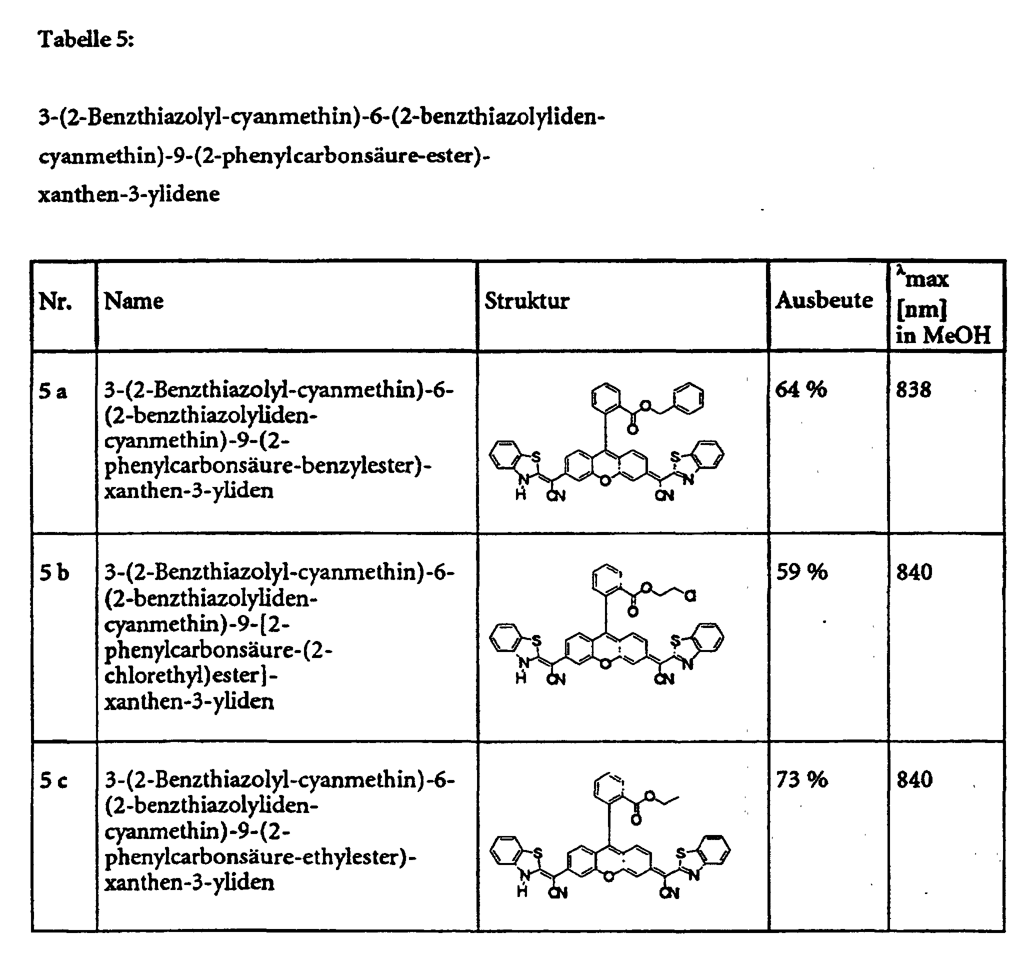

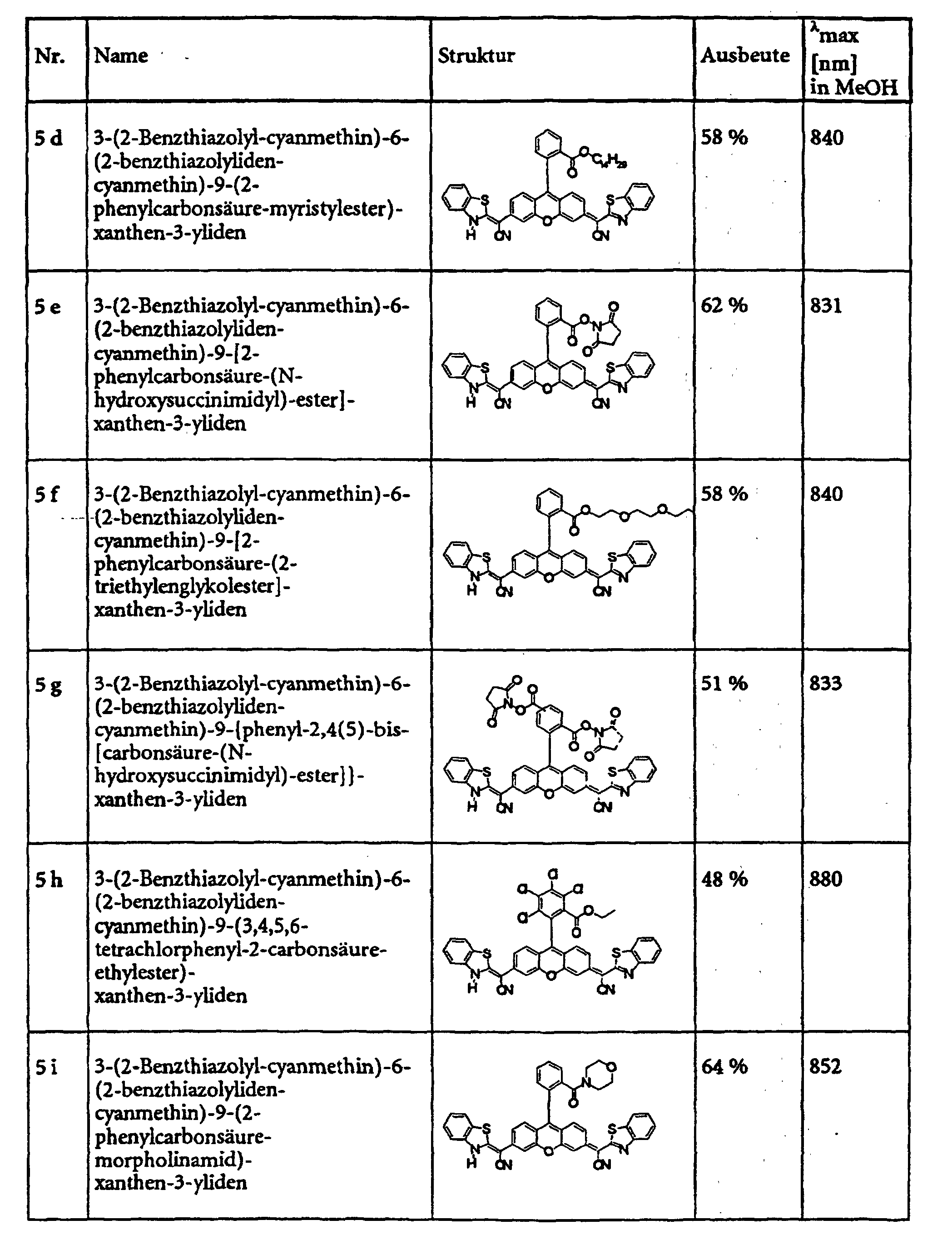

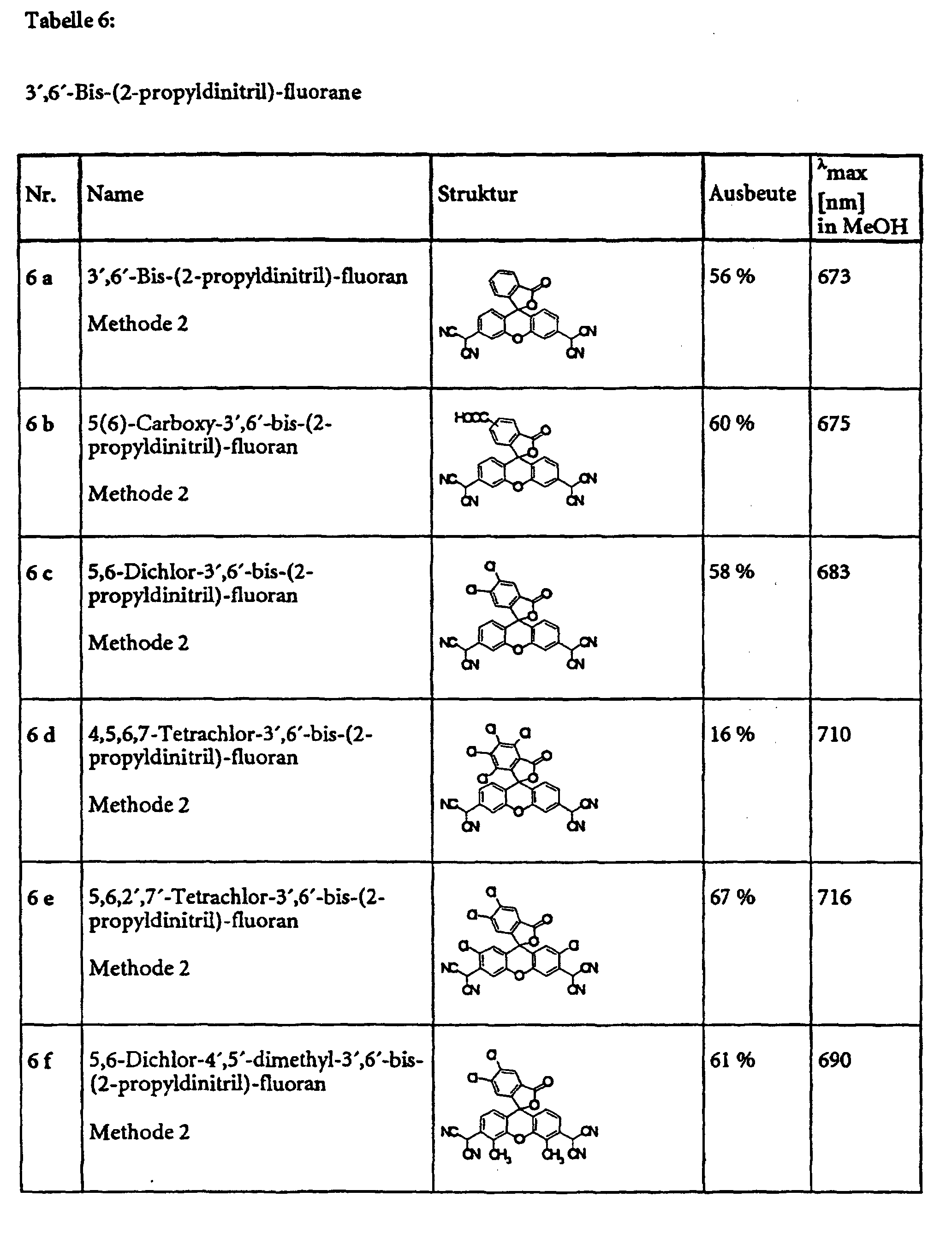

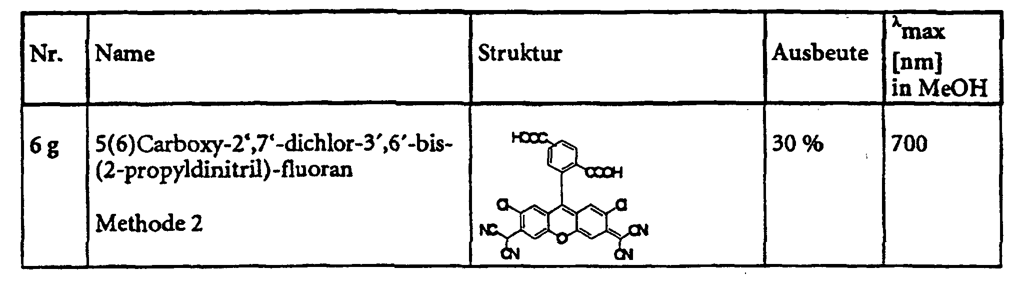

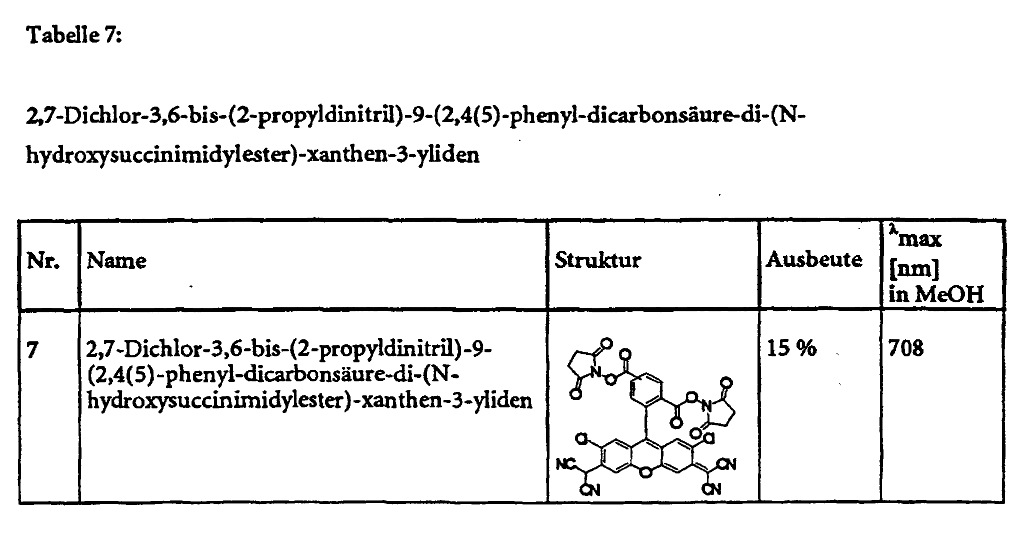

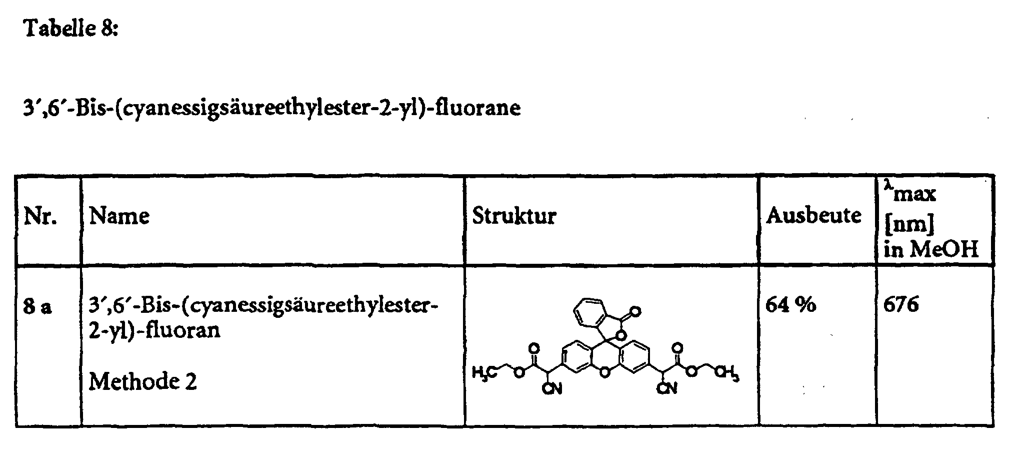

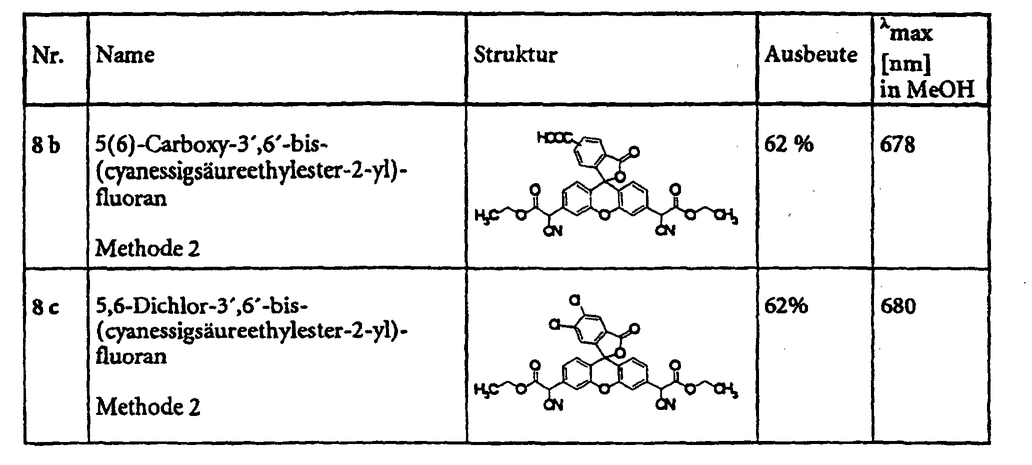

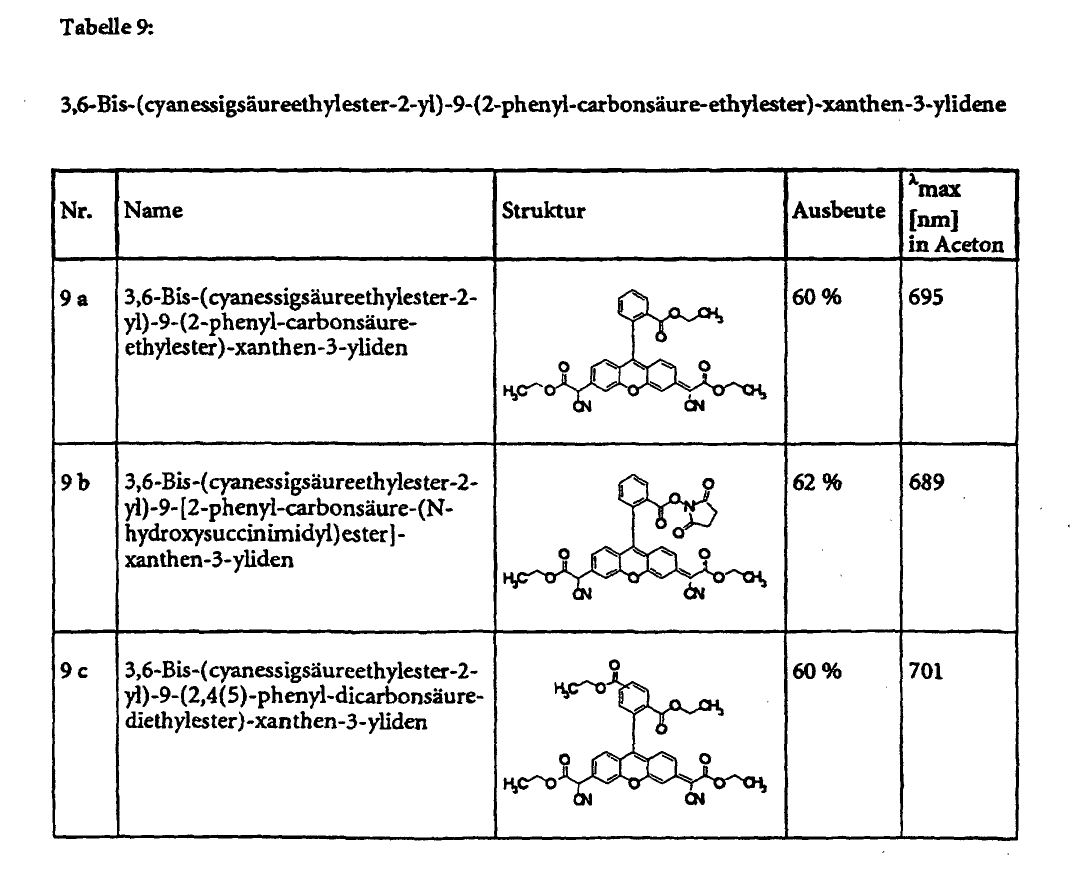

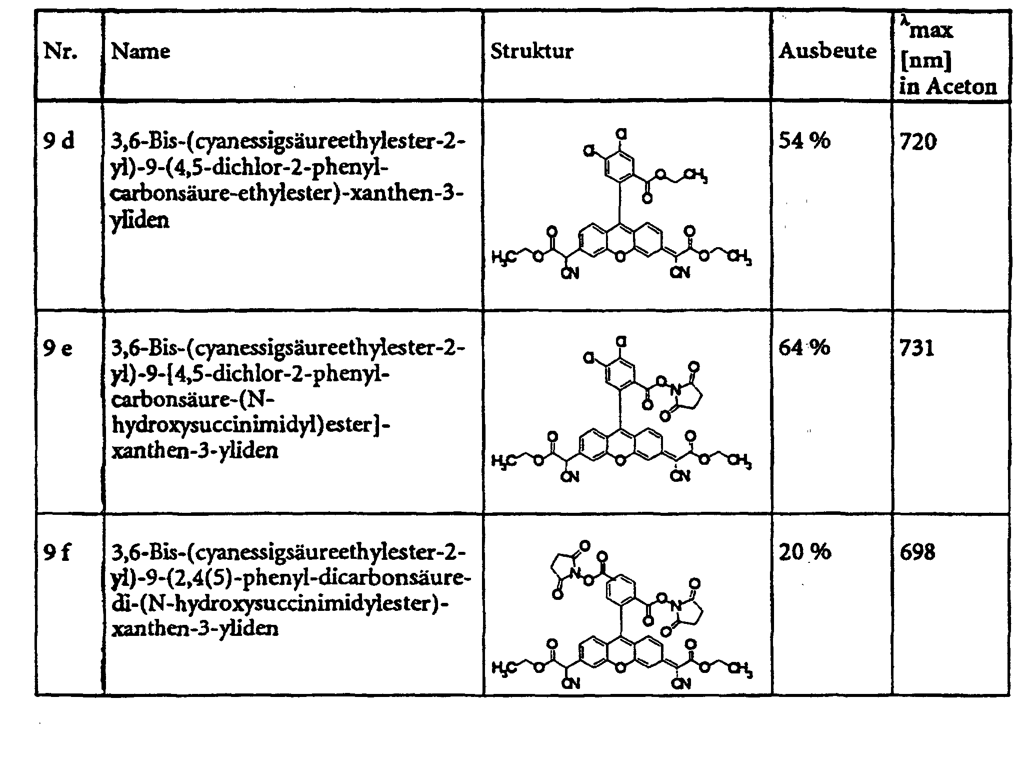

## Patentansprüche

1. Xanthenderivate der allgemeinen Formel I 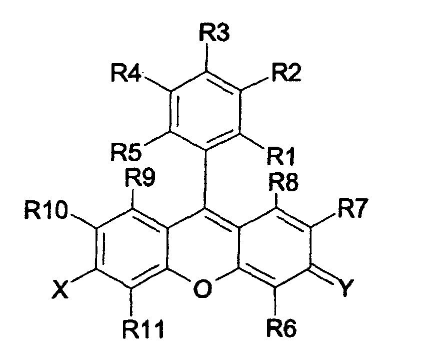 worin R1,R2, R3, R4, R5 unabhängig voneinander Wasserstoff, Alkyl, Aryl, Heteroaryl, Alkoxy, Cyano, Hydroxy, Halogen, Isocyanat, Isothiocyanat oder Carboxyl, Sulfonyl, bzw. Derivate davon wie Alkoxycarbonyl, Aryloxycarbonyl, Aktivester, Säurehalogenide, gemischte Anhydride oder unsubstituierte oder substituierte Aminoreste, speziell ω-Aminoalkohole, ω-Aminocarbonsäuren oder ω-Aminohalogenalkane sein können;
es sich bei den Resten R6, R7, R8, R9, R10, R11 unabhängig voneinander um Wasserstoff, Alkyl- , substituierte Alkylreste oder Halogenid, insbesonders Wasserstoff- oder Alkylreste mit 1-10 C-Atomen, oder Sulfoalkylreste, insbesonders Sulfomethylreste oder Chlor bzw. Fluor handelt,
wobei X und Y ein Amin oder ein substituiertes Amin,
oder einen elektronenziehenden Rest aus den Gruppen
a) der Aryliden- bzw. Heteroarylidencyanmethine, z.B. 2-Benzthiazolylidencyanmethine oder 2- bzw. 4-Chinolinylidencyanmethine oder
b) der Malonsäurederivate, z.B. 2-Propyldinitril-Resten oder Cyanoessigsäureester
repräsentieren und wobei mindestens einer der Reste X oder Y eine der besagten elektronenziehenden Gruppen darstellt.

2. Xanthenderivat gemäß Anspruch 1, **dadurch gekennzeichnet, daß** X gleich Y ist.

3. Xanthenderivat gemäß Anspruch 1, **dadurch gekennzeichnet, daß** X ungleich Y ist.

4. Xanthenderivat gemäß Anspruch 1 oder 3, **dadurch gekennzeichnet, daß** X oder Y einen Amin-, bzw. substituierten Amin-Rest bedeutet, und Y oder X jeweils einen elektronenziehenden Rest repräsentiert.

5. Xanthenderivate gemäß Anspruch 4, **dadurch gekennzeichnet, daß** X oder Y ein Alkyl-substituiertes oder cyclisches Amin bedeutet.

6. Xanthenderivate gemäß Anspruch 1 bis 5, **dadurch gekennzeichnet, daß** mindestens einer der Reste R1 bis R11 eine aktivierbare Gruppe, z.B. eine Carbonsäure oder Sulfonsäuregruppe darstellt oder eine aktivierte Gruppe, z.B. ein(en) Säureester, Säureanhydrid, Säurehalogenid oder N-Hydroxysuccinimidester darstellt.

7. Xanthenkonjugat bestehend aus einem Xanthenderivat nach Ansprüchen 1 - 6 sowie mindestens einem Biomolekül.

8. Xanthenkonjugat gemäß Anspruch 1 bis 7, **dadurch gekennzeichnet, daß** die biologisch aktive Gruppe ein Hapten, Antigen, Antikörper oder Protein darstellt.

9. Xanthenkonjugat gemäß Anspruch 1-7, **dadurch gekennzeichnet, daß** das Biomolekül ein Mono- oder Polynukleotid darstellt.

10. Verfahren zur Bestimmung einer Substanz, bei welchem die spezifische Binderreaktion an diese Substanz über Veränderungen der Absorption, Fluoreszenz oder Fluoreszenzpolarisation ermittelt wird, **dadurch gekennzeichnet, daß** ein Konjugat gemäß Anspruch 7 oder 8 eingesetzt wird.

11. Verwendung eines Konjugates gemäß Anspruch 8 für diagnostische Verfahren.

12. Verwendung eines Konjugates gemäß Anspruch 9 in der Nucleinsäure-Analytik.

13. Verwendung der Konjugate gemäß Anspruch 8 oder 9 in Energy-Transfer Systemen.

14. Verwendung der Xanthenderivate gemäß Anspruch 1 bis 6 zum Färben von Latexpartikeln.

15. Verwendung der Xanthenderivate gemäß Anspruch 1 bis 6 als in vivo-Farbstoffe.

16. Verwendung der Xanthenderivate gemäß Anspruch 1 bis 6 als Laserfarbstoffe.

17. Verfahren zur Herstellung der Xanthenderivate der Formel I gemäß Anspruch 1, **dadurch gekennzeichnet, daß** 3',6'-abgangsgruppensubstituierte Xanthene oder Fluorane mit C-Nukleophilen (aus CH-aciden Verbindungen) umgesetzt werden.

## Claims

1. Xanthene derivatives of the general formula I 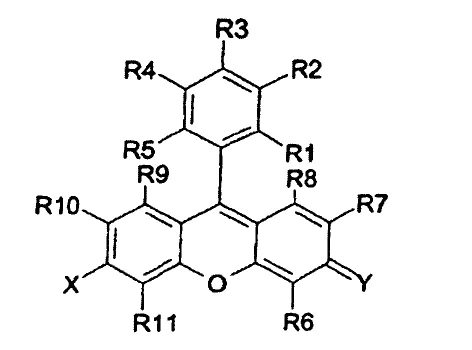 wherein R1, R2, R3, R4, R5 can, independently of one another, be hydrogen, alkyl, aryl, heteroaryl, alkoxy, cyano, hydroxy, halogen, isocyanate, isothiocyanate or carboxyl, sulfonyl or derivatives thereof such as alkoxycarbonyl, aryloxycarbonyl, active ester, acid halogenides, mixed anhydrides or unsubstituted or substituted amino residues, especially ω-amino alcohols, ω-aminocarboxylic acids or ω-aminohalogenalkanes;
the residues R6, R7, R8, R9, R10, R11 are, independently of one another, hydrogen, alkyl, substituted alkyl residues or halogenide, and in particular hydrogen or alkyl residues with 1-10 C atoms or sulfoalkyl residues, especially sulfomethyl residues, or chlorine or fluorine, respectively,
wherein X and Y represent an amine or a substituted amine or an electrophilic residue from the following groups
a) arylidene or heteroarylidenecyanomethines such as 2-benzthiazolylidenecyanomethines or 2- or 4-quinolinylidenecyanomethines or
b) malonic acid derivatives such as 2-propyldinitrile residues or cyanoacetic acid esters.
and wherein at least one of the residues X or Y represents one of the said electrophilic groups.

2. Xanthene derivative as claimed in claim 1, **characterized in that** X is identical to Y.

3. Xanthene derivative as claimed in claim 1, **characterized in that** X and Y are different.

4. Xanthene derivative as claimed in claim 1 or 3, **characterized in that** X or Y denotes an amine or substituted amine residue and Y or X each represent an electrophilic residue.

5. Xanthene derivatives as claimed in claim 4, **characterized in that** X or Y denotes an alkyl-substituted or cyclic amine.

6. Xanthene derivatives as claimed in claims 1 to 5, **characterized in that** at least one of the residues R1 to R11 is a group that can be activated e.g. a carboxylic acid or sulfonic acid group or an activated group e.g. an acid ester, acid anhydride, acid halogenide or N-hydroxysuccinimide ester.

7. Xanthene conjugate consisting of a xanthene derivative as claimed in claims 1 - 6 and at least one biomolecule.

8. Xanthene conjugate as claimed in claims 1 to 7, **characterized in that** the biologically active group is a hapten, antigen, antibody or protein.

9. Xanthene conjugate as claimed in claims 1 - 7, **characterized in that** the biomolecule is a mononucleotide or polynudeotide.

10. Method for determining a substance in which the specific binding reaction to this substance is determined by changes in absorption, fluorescence or fluorescence polarization, **characterized in that** a conjugate as claimed in claim 7 or 8 is used.

11. Use of a conjugate as claimed in claim 8 for diagnostic methods.

12. Use of a conjugate as claimed in claim 9 in nucleic acid analytics.

13. Use of the conjugates as claimed in claim 8 or 9 in energy transfer systems.

14. Use of the xanthene derivatives as claimed in claims 1 to 6 to dye latex particles.

15. Use of the xanthene derivatives as claimed in claims 1 to 6 as in vivo dyes.

16. Use of the xanthene derivatives as claimed in claims 1 to 6 as laser dyes.

17. Process for producing the xanthene derivatives of formula I as claimed in claim 1, **characterized in that** xanthenes or fluorans that are 3', 6'-substituted with leaving groups are reacted with C-nucleophiles (from CH acidic compounds).

## Revendications

1. Dérivés du xanthène de formule générale I 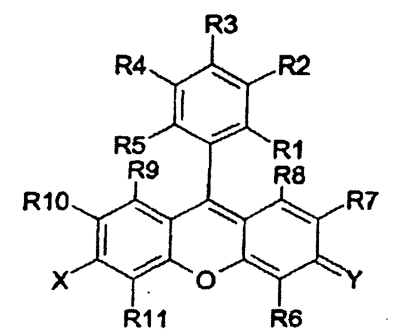 dans laquelle R1, R2, R3, R4, R5 peuvent représenter indépendamment l'un de l'autre un atome d'hydrogène, un groupe alkyle, un groupe aryle, un groupe hétéroaryle, un groupe alcoxy, un groupe cyano, un groupe hydroxy, un atome d'halogène, un groupe isocyanate, un groupe isothiocyanate ou un groupe carboxyle, un groupe sulfonyle ou des dérivés de ceux-ci, tels qu'un groupe alcoxycarbonyle, un groupe aryloxycarbonyle, des esters actifs, des halogénures d'acide, des anhydrides mixtes ou des radicaux amino non substitués ou substitués, en particulier des ω-aminoalcools, des acides ω-aminocarboxyliques ou des ω-aminohalogénoalcanes;
dans laquelle il s'agit, pour les radicaux R6, R7, R8, R9, R10, R11, indépendamment l'un de l'autre, d'un atome d'hydrogène, de radicaux alkyle, de radicaux alkyle substitués ou d'un halogénure, en particulier des atomes d'hydrogène ou des radicaux alkyle comprenant 1 à 10 atomes de C ou des radicaux sulfoalkyle, en particulier des radicaux sulfométhyle, ou de chlore ou de fluor,
X et Y représentant une amine ou une amine substituée
ou un radical attirant les électrons parmi les groupes
a) des arylidènecyanométhines ou des hétéroarylidènecyanométhines, par exemple la 2-benzthiazolylidènecyanométhine ou la 2-quinoléinylidènecyanométhine ou la 4-quinoléinylidènecyanométhine ou
b) des dérivés de l'acide malonique, par exemple des radicaux 2-propyldinitrile
ou des esters de l'acide cyanoacétique
et au moins un des radicaux X ou Y représente un des groupes attirant les électrons mentionnés.

2. Dérivé du xanthène selon la revendication 1, **caractérisé en ce que** X est identique à Y.

3. Dérivé du xanthène selon la revendication 1, **caractérisé en ce que** X est différent de Y.

4. Dérivé du xanthène selon la revendication 1 ou 3, **caractérisé en ce que** X ou Y signifie un radical amine ou un radical amine substituée et Y ou X représentent à chaque fois un radical attirant les électrons.

5. Dérivés du xanthène selon la revendication 4, **caractérisés en ce que** X ou Y signifie une amine substituée par un groupe alkyle ou une amine cyclique.

6. Dérivés du xanthène selon les revendications 1 à 5, **caractérisés en ce qu'**au moins un des radicaux R1 à R11 représente un groupe activable, par exemple un groupe acide carboxylique ou un groupe acide sulfonique, ou un groupe activé, par exemple un ester d'acide, un anhydride d'acide, un halogénure d'acide ou un ester N-hydroxysuccinimidique.

7. Conjugué du xanthène, constitué d'un dérivé du xanthène selon les revendications 1 à 6 ainsi que d'au moins une biomolécule.

8. Conjugué du xanthène selon les revendications 1 à 7, **caractérisé en ce que** le groupe biologiquement actif est un haptène, un antigène, un anticorps ou une protéine.

9. Conjugué du xanthène selon les revendications 1 à 7, **caractérisé en ce que** la biomolécule représente un mononucléotide ou un polynucléotide.

10. Procédé pour la détermination d'une substance, dans lequel la réaction de fixation spécifique sur cette substance est déterminée via des modifications de l'absorption, de la fluorescence ou de la polarisation de la fluorescence, **caractérisé en ce qu'**on utilise un conjugué selon la revendication 7 ou 8.

11. Utilisation d'un conjugué selon la revendication 8 pour des procédés diagnostiques.

12. Utilisation d'un conjugué selon la revendication 9 dans l'analyse des acides nucléiques.

13. Utilisation des conjugués selon la revendication 8 ou 9 dans des systèmes de transfert d'énergie.

14. Utilisation des dérivés du xanthène selon les revendications 1 à 6 pour la coloration de particules de latex.

15. Utilisation des dérivés du xanthène selon les revendications 1 à 6 comme colorants in vivo.

16. Utilisation des dérivés du xanthène selon les revendications 1 à 6 comme colorants laser.

17. Procédé pour la préparation des dérivés du xanthène de formule I selon la revendication 1, **caractérisé en ce qu'**on transforme des xanthènes substitués en positions 3', 6' par des groupes sortants ou des fluoranes avec des atomes de C nucléophiles (à partir de composés à CH acide).
